## Europäisches Patentamt
## European Patent Office
## Office européen des brevets

(19)

(11) Publication number: **0 032 807**
**B1**

(12) **EUROPEAN PATENT SPECIFICATION**

(45) Date of publication of patent specification: **19.01.83**

(21) Application number: **81300139.3**

(22) Date of filing: **14.01.81**

(51) Int. Cl.³: **C 07 C 53/122,**
**C 07 C 51/41**

(54) Dipotassium pentapropionate and method of preparing the same.

(30) Priority: **17.01.80 GB 8001543**

(43) Date of publication of application:
**29.07.81 Bulletin 81/30**

(45) Publication of the grant of the patent:
**19.01.83 Bulletin 83/3**

(84) Designated Contracting States:
**BE DE FR GB IT NL SE**

(56) References cited:
**DE - C - 424 017**
**DE - C - 932 607**
**US - A - 3 919 307**

(73) Proprietor: **BP Chemicals Limited**
**Britannic House Moor Lane**
**London, EC2Y 9BU (GB)**

(72) Inventor: **Brankling, David**
**BP Chemicals Limited Salt End Hedon**
**Hull HU12 8DS (GB)**

(74) Representative: **Harry, John et al,**
**BP INTERNATIONAL LIMITED Patents and**
**Licensing Division Chertsey Road**
**Sunbury-on-Thames Middlesex TW16 7LN (GB)**

Courier Press, Leamington Spa, England.

Dipotassium pentapropionate and method of preparing the same

The present invention relates to solid acid potassium propionates and a process for preparing the same.

Acid salts formed from lower monocarboxylic acids and alkali metals are well known. Aqueous solutions of such acid salts are claimed and described in our British Patent Specification No: 1505388. Some of these acid salts have also been prepared in their solid form but procedures used to isolate the solids are rather complicated due to the relative instability of the salts. For example, anhydrous conditions are used in the process described by Becker et al in J.A.C.S., volume 85 (1963), pp 157—159.

A method of producing some of these acid salts from aqueous solutions is disclosed in Deutsche Offenlegungsschrift 2432473. However, the technique used is complicated in that a fluidised bed and an inert atmosphere are necessary to obtain the desired product.

The acid salts are very versatile and have immense commercial potential due to their reduced odour and corrosivity in comparison with free acids and their greater activity in relation to the neutral salts. For example, compositions containing these acid salts may be used as fungicides, mould inhibitors, crop preservatives, bread preservatives, setting accelerators for concrete, disinfectants and as dyeing auxiliaries. However, the difficulties encountered in producing relatively stable acid salts in a solid form necessitate the use of these salts as solutions. For some uses, e.g. preservation of compound animal feedstuffs, solid preservatives are preferred, because of the types of material, machinery and processing involved.

It is an object of the present invention to produce a stable, solid acid salt.

Accordingly, the present invention is solid dipotassium pentapropionate.

According to a further embodiment the present invention is a process of producing dipotassium pentapropionate which comprises cooling an aqueous solution of potassium dipropionate of a concentration of at least 50% w/w to a temperature at which dipotassium pentapropionate crystallises and recovering the solid, crystalline dipotassium pentapropionate.

The starting material for the production of dipotassium pentapropionate according to the present invention is potassium dipropionate. Potassium dipropionate may be produced by reacting neutral potassium propionate with free propionic acid. The neutral potassium propionate may be in the form of solid salt or in aqueous solution. Alternatively, potassium hydroxide or potassium carbonate may be reacted with free propionic acid. The hydroxide or carbonate may be in the form of solids or in aqueous solutions. Whichever base is used it is reacted with an excess of propionic acid so as to form a solution of potassium dipropionate from which dipotassium pentapropionate is crystallised according to the process of the present invention.

It is however preferable to form the potassium dipropionate by reacting the neutral salt with free propionic acid. This may be achieved by first reacting an aqueous solution of potassium hydroxide with propionic acid to obtain an aqueous solution of the neutral propionate. Water may then be evaporated from this solution, if necessary under reduced pressure, e.g. by heating at 80°C and 130 m bar pressure to form a concentrated solution of neutral potassium propionate. The additional propionic acid needed to form the potassium dipropionate is then added in the appropriate stoichiometric amount to the concentrated solution of the neutral salt.

The concentration of the potassium dipropionate solution from which the dipotassium pentapropionate is to be crystallised is suitably between 60 and 95 % w/w, preferably between 70 and 90 % w/w. The solution may contain more or less than the stoichiometric proportion of propionic acid required to convert the neutral propionate to the dipropionate.

In carrying out the process of the invention the solution of potassium dipropionate is cooled to a temperature at which dipotassium pentapropionate crystallises out. The crystallisation temperature will vary depending on the concentration of the potassium dipropionate solution, and on the relative proportions of potassium dipropionate, potassium neutral propionate and propionic acid. Thus where the potassium dipropionate solution contains more than the theoretical amount of propionic acid required to form the dipropionate, crystallisation of the dipotassium pentaproprionate will occur at temperatures above 0° C, e.g. at about 5° C. On the other hand if the potassium dipropionate starting solution contains less than the theoretical amount of propionic acid required to form the dipropionate it is necessary to cool the solution to below 0° C for crystallisation of the pentapropionate to occur. Where the concentration of the potassium dipropionate solution does not exceed 75% w/w, crystallisation of the dipotassium pentapropionate will occur directly, on cooling to the appropriate temperature. However on cooling potassium dipropionate solutions of concentrations exceeding 75% w/w crystals of potassium dipropionate initially separate out as platelets at ambient temperature and can be removed by filtration. On further cooling crystals of varying composition between the dipropionate and the pentapropionate separate out and again can be removed by filtration. On still further cooling pure dipotassium pentapropionate crystals separate out in the form of white needles.

Precipitation of the desired dipotassium pentapropionate crystals may be facilitated by 'seeding' the solution with crystals of the pentapropionate.

Yet another method of producing dipotassium pentapropionate comprises reacting solid potassium dipropionate, e.g. separated as a by-product during the various crystallisation stages, with an appropriate stoichiometric amount of propionic acid in aqueous solution and finally cooling the reaction solution to ambient temperature allowing solid dipotassium pentapropionate to separate.

Dipotassium pentapropionate can also be produced in amorphous non-crystalline form by reacting neutral potassium propionate with the stoichiometric amount of free propionic acid.

The solid dipotassium pentapropionate can be represented by the molecular formula:

$$(KO\ CO\!-\!\!-\!CH_2CH_3)_2.3CH_3.CH_2COOH$$

and is in the form of white crystalline needles or white amorphous powder, with a titratable propionic acid content of $49.8 \pm 0.6\%$ w/w, a melting point at 94° C and a solubility of 80% w/w in water at room temperature.

The invention is further illustrated with reference to the following examples:

### Example 1

198.9g of propionic acid was added to 301g of a 50% aqueous potassium hydroxide solution, to produce the neutral salt. The temperature was raised to 80—90° C and pressure reduced to 130 Mbar. Water was stripped from the solution until the neutral salt began to crystallise at the stated temperature (80—90° C). 198.9g of propionic acid was then added to produce an 90% w/w solution of the dipropionate.

On cooling to 20° C, 172g of platelets of potassium dipropionate containing 41.0% w/w titratable acid were obtained, and this was separated from the mother liquor by filtration.

Further cooling of the resultant filtrate to 0° C produced 71g of needles containing 45.0% w/w titratable acid representing mixed potassium dipropionate/dipotassium pentapropionate. This was again separated by filtration.

On cooling the filtrate to —5° C approximately 15g of needles of 49.8% w/w titratable acid separated representing pure dipotassium pentapropionate.

### Example 2

11.2g of solid potassium hydroxide was added to 30g of propionic acid such that a 91% solution of potassium dipropionate was produced. Cooling of this solution to 20° C precipitated 2.3g of potassium dipropionate crystals of 39.7% w/w titratable acidity. Further cooling to successively low temperatures as in Example 1 above gave the following products:

i) platelets at 11° C of 42.7% w/w titratable acid

ii) needles at 5° C of 47.0% w/w titratable acid

iii) needles at —5° C of 49.2% w/w titratable acid representing essentially pure dipotasium pentapropionate.

### Example 3

56g of a 50% w/w aqueous potassium hydroxide was reacted with 74g of propionic acid to produce a 71.5% w/w solution of potassium dipropionate. Cooling of this to 5° C produced no crystals. Cooling to —5° C produced 18g of needles of pure dipotassium pentapropionate equivalent to 49.8 w/w titratable acid.

### Example 4

15.0g of propionic acid was added to 13.8g of anhydrous potassium carbonate and the resultant solution dried at 140° C to produce 22.4g of the anhydrous neutral salt. 22.2g of propionic acid were then added to this neutral salt to produce 44.6g of solid amorphous dipotassium pentapropionate which could be ground to a fine free flowing powder of 49.8% w/w titratable acidity.

### Example 5

5.6g of solid potassium hydroxide were reacted with 18.5g of propionic acid to produce a solution of potassium dipropionate containing excess of propionic acid. Cooling of the solution to 25° C precipitated 2.8g of a mixture of the dipropionate and pentapropionate of 47.0% titratable acidity. Filtration followed by further cooling to 5° C precipitated 5g of pure dipotassium pentapropionate equivalent to 49.8% w/w titratable acid.

### *Fungicidal Activity of Dipotassium Pentapropionate*

Samples of potassium dipropionate and dipotassium pentapropionate were included in samples of a malt extract medium at concentrations of 0.1 and 0.25% by weight. The resulting plates were stab inoculated with *Aspergillus flavus* and *Penicillium notatum* and the colony diameters measured after incubation for 2, 3 and 6 days at 27° C. The results are presented below:

| Inhibitor | Average Colony Diameter (mm) |
|---|---|
| 0.1% Potassium dipropionate | 21.2 |
| 0.25% Potassium dipropionate | 11.5 |
| 0.1% Dipotassium pentapropionate | 19.4 |
| 0.25% Dipotassium pentapropionate | 7.6 |

The results clearly indicate that the dipotassium pentapropionate has a higher fungicidal activity than potassium dipropionate.

## Claims

1. Solid dipotassium pentapropionate.

2. Solid, crystalline dipotassium pentapropionate.

3. The process of producing solid dipotassium pentapropionate, which comprises cooling an aqueous solution of potassium dipropionate of a concentration of at least 50% w/w to a temperature at which dipotassium pentapropionate crystallises and recovering the solid, crystalline dipotassium pentapropionate.

4. A process according to claim 3 wherein the solution of potassium dipropionate is produced by reacting neutral potassium propionate with propionic acid.

5. A process according to either of the preceding claims 3 or 4 in which the concentration of the solution of potassium dipropionate is between 60 and 95% w/w.

6. A process according to any of the preceding claims 3 to 5 in which the concentration of the solution of potassium dipropionate is between 70 to 90% w/w.

7. A method of producing solid crystalline dipotassium pentapropionate which comprises cooling a solution of potassium dipropionate of a concentration exceeding 75% w/w to a temperature at which crystals of potassium dipropionate separate, removing the crystals of potassium dipropionate from the mother liquor, further cooling the mother liquor to a temperature at which crystals of dipotassium pentapropionate separate, and recovering the crystalline dipotassium pentapropionate.

8. A method of producing solid crystalline dipotassium pentapropionate, said method comprising reacting a solution of neutral potassium propionate with propionic acid, the relative amounts of the reactants being such that the theoretical concentration of the potassium dipropionate in the reaction solution does not exceed 75% w/w, cooling the solution to a temperature at which crystalline dipotassium pentapropionate separates and recovering said dipotassium pentapropionate.

9. A method of producing solid dipotassium pentapropionate, said method comprising reacting neutral potassium propionate with the stoichiometric amount of propionic acid required to form solid dipotassium pentapropionate.

## Revendications

1. Pentapropionate de dipotassium solide.

2. Pentapropionate de dipotassium cristallin solide.

3. Procédé de production de pentapropionate de dipotassium solide, qui comprend le refroidissement d'une solution aqueuse de dipropionate de potassium d'une concentration d'au moins 50 % en poids/poids, jusqu'à une température à laquelle le pentapropionate de dipotassium cristallise, et la récupération du pentapropionate de dipotassium cristallin solide.

4. Procédé la revendication 3, dans lequel la solution de dipropionate de potassium est produite par la réaction de propionate de potassium neutre avec de l'acide propionique.

5. Procédé selon l'une ou l'autre des revendications 3 ou 4 précédentes, dans lequel la concentration de la solution de dipropionate de potassium se situe entre 60 et 95 % en poids/poids.

6. Procédé selon l'une quelconque des revendications 3 à 5 précédentes, dans lequel la concentration de la solution de dipropionate de potassium se situe entre 70 et 90 % en poids/poids.

7. Procédé de production de pentapropionate de dipotassium cristallin solide, qui consiste à refroidir une solution de dipropionate de potassium, d'une concentration excédant 75 % en poids/poids, jusqu'à une température à laquelle des cristaux de dipropionate de potassium se séparent, à enlever les cristaux de dipropionate de potassium de la liqueur mère, à refroidir encore la liqueur mère jusqu'à une température à laquelle des cristaux de pentapropionate de dipotassium se séparent, et à récupérer le pentapropionate de dipotassium cristallin.

8. Procédé de production de pentapropionate de dipotassium cristallin solide, ce procédé comprenant la réaction d'une solution de propionate de potassium neutre avec de l'acide propionique, les quantités relatives des corps mis en réaction étant telles que la concentration théorique du dipropionate de potassium dans la solution de réaction n'excède pas 75 % en poids/poids, le refroidissement de la solution jusqu'à une température à laquelle du pentapropionate de dipotassium cristallin se sépare et la récupération de ce pentapropionate de dipotassium.

9. Procédé de production de pentapropionate de dipotassium solide, ce procédé comprenant la réaction du propionate de potassium neutre avec la quantité stoéchiométrique d'acide propionique nécessaire pour former du pentapropionate de dipotassium solide.

## Patentansprüche

1. Festes Dikaliumpentapropionat.

2. Festes, kristallines Dikaliumpentapropionat.

3. Verfahren zur Herstellung von festem Dikaliumpentapropionat, welches umfaßt: Kühlen einer wässrigen Lösung von Kaliumdipropionat mit einer Konzentration von mindestens 50 Gew./Gew.-% auf eine Temperatur, bei der Dikaliumpentapropionat kristallisiert und Gewinnung des festen kristallinen Dikaliumpentapropionats.

4. Verfahren nach Anspruch 3, wonach die Lösung von Kaliumdipropionat durch Umsetzung von neutralem Kaliumpropionat mit Propionsäure hergestellt worden ist.

5. Verfahren nach Anspruch 3—4, wonach die Konzentration der Lösung von Kaliumdipropionat zwischen 60 und 95 Gew./Gew.-% beträgt.

6. Verfahren nach Anspruch 3—5, worin die Konzentration der Lösung von Kaliumdipropionat zwischen 70 und 90 Gew./Gew.-% beträgt.

7. Verfahren zur Herstellung festen, kristallinen Dikaliumpentapropionats, welches umfaßt: Kühlen einer Lösung von Kaliumdipropionat mit einer Konzentration von mehr als 75 Gew./Gew.-% auf eine Temperatur, bei der sich Kaliumdipropionat-Kristalle abscheiden, Entfernen der Kaliumdipropionat-Kristalle aus der Mutterlauge, weiteres Abkühlen der Mutterlauge auf eine Temperatur, bei der sich Dikaliumpentapropionat-Kristalle abscheiden und Gewinnung des kristallinen Dikaliumpentapropionats.

8. Verfahren zur Herstellung kristallinen Dikaliumpentapropionats, welches umfaßt: Umsetzen einer Lösung von neutralem Kaliumpropionat mit Propionsäure, wobei die relativen Mengen der Reactionsteilnehmer derart sind, daß die theoretische Konzentration des Kaliumdipropionats in der Reaktionslösung 75 Gew./Gew.-% nicht übersteigt, Kühlen der Lösung auf eine Temperatur, bei der sich kristallines Dikaliumpentapropionat abscheidet und Gewinnung des Dikaliumpentapropionats.

9. Verfahren zur Herstellung festen Dikaliumpentapropionats, welches umfaßt: Umsetzen neutralen Kaliumpropionats mit den stöchiometrischen Mengen an Propionsaüre, die erforderlich sind, festes Dikaliumpentapropionat zu bilden.